# DEMANDE DE BREVET EUROPEEN

(11) **EP 4 311 808 A1**
(43) Date de publication de la demande: **31.01.2024**
(21) Numéro de dépôt: 23188299.4
(22) Date de dépôt: 28.07.2023
(51) Int. Cl.: C01G 49/08, G01N 27/74, G01R 33/12

(54) **NANO- OU MICROPARTICULE COMPRENANT UNE MATRICE D ALCOOL POLYVINYLIQUE, ET, EN DISPERSION DANS CETTE DERNIERE, DE LA FERRITE, SON PROCEDE D OBTENTION ET SES UTILISATIONS**

(30) Priorité: 28.07.2022 FR 2207815
(71) Demandeur: Commissariat à l'énergie atomique et aux énergies alternatives, 75015 Paris (FR)
(72) Inventeur: PONCELET, Olivier, 38054 GRENOBLE cedex 09 (FR); DURET, Antonin, 91191 GIF SUR YVETTE CEDEX (FR); JASMIN-LEBRAS, Guénaëlle, 91191 GIF-SUR-YVETTE CEDEX (FR); DEROO, Maïkane, 91191 GIF-SUR-YVETTE CEDEX (FR); FERAUDET-TARISSE, Cécile, 91191 GIF-SUR-YVETTE CEDEX (FR)
(74) Mandataire: Gevers & Orès

(57) **Abrégé**

La présente invention concerne une nano- ou microparticule comprenant une matrice constituée de ou comprenant au moins un alcool polyvinylique (PVA), et, en dispersion dans cette dernière, de la ferrite, ainsi que son procédé d'obtention. La présente invention vise également l'utilisation de ces nano- ou microparticules pour la préparation et la mise en oeuvre de dispositifs susceptibles d'être détectés par des capteurs à magnétorésistance géante (capteurs GMR) en tant qu'outils de diagnostic biologique.

## Description

La présente invention concerne une nano- ou microparticule comprenant une matrice constituée de ou comprenant au moins un alcool polyvinylique (PVA), et, en dispersion dans cette dernière, de la ferrite, ainsi que son procédé d'obtention. La présente invention vise également l'utilisation de ces nano- ou microparticules pour la préparation et la mise en oeuvre de dispositifs susceptibles d'être détectés par des capteurs à magnétorésistance géante (capteurs GMR) en tant qu'outils de diagnostic biologique.

Le diagnostic clinique est un domaine dans lequel de nouvelles méthodes d'analyse plus rapides, directes, précises, sélectives, de surcroît à haut rendement et bon marché, sont très demandées. En raison de leur petite taille, de leur transduction ultra-sensible et de leur intégration possible dans des systèmes de type « laboratoire sur puce », les dispositifs de biodétection fabriqués à l'aide des nanotechnologies sont un candidat potentiel pour répondre à toutes les exigences ci-dessus. Depuis la dernière décennie, de nombreux travaux sur le biomagnétisme et les biocapteurs magnétiques, basés sur des processus moléculaires, ont été réalisés, notamment sur l'application des nanoparticules magnétiques en biomédecine, sur leur synthèse, leur fonctionnalisation et leur détection par des capteurs magnétiques.

Les capteurs magnétorésistifs semblent être parmi les meilleurs candidats pour répondre à ces critères. Depuis la fin des années 1990, les capteurs à magnétorésistance géante (capteurs GMR) ultra sensibles sont d'excellents candidats pour la détection magnétique dans des domaines très variés dont celui de la santé. Ce sont des capteurs très sensibles à base d'électronique de spin. Ils présentent l'avantage d'avoir une très grande détectivité (la détectivité correspond au champ magnétique pour lequel le rapport signal sur bruit vaut 1 ; elle s'exprime en T/√Hz, et permet une comparaison aisée de performances des différents capteurs magnétiques), de l'ordre de 50 à 200 pT/√Hz, d'être peu coûteux et de pouvoir être intégrés facilement dans des laboratoires sur puce.

Dans ce domaine, les cibles biologiques sont marquées magnétiquement par des nano- ou microparticules magnétiques fonctionnalisées avec des anticorps spécifiques dirigés contre la cible biologique d'intérêt. Ces objets marqués magnétiquement sont alors détectés de manière dynamique (ou statique) par les capteurs GMR. Cette technologie est basée sur la détection de marqueurs magnétiques

Ces dernières années, les capteurs GMR ont montré un grand potentiel comme capteurs pour la détection de cibles biologiques (cellules ou bactéries). La résistance d'un capteur GMR varie en fonction du champ magnétique appliqué au capteur, de sorte qu'un objet biologique marqué magnétiquement peut induire un signal. Par rapport à la détection optique traditionnelle, largement utilisée en biomédecine, les capteurs GMR permettent de travailler avec des matrices complexes, même opaques, et permettent la détection dynamique des objets magnétiques un à un. D'autre part, une nouvelle biopuce, à base de capteurs GMR disposés de part et d'autre du canal microfluidique, permet de déterminer le moment magnétique des objets détectés et donc d'identifier un grand nombre des faux positifs provenant de la présence d'agrégats de nanoparticules magnétiques (brevet FR3082620). Cependant, le verrou à lever pour augmenter encore la sensibilité d'une telle technique de détection est de diminuer le nombre de ces agrégats de nanoparticules magnétiques qui se confondent encore avec les cibles biologiques d'intérêt marquées

En raison des avantages des matériaux GMR pour les mesures de champ magnétique, tels que la haute sensibilité et la réponse rapide sous un faible champ magnétique, une attention accrue a été accordée au développement de ces matériaux GMR pour les biocapteurs.

L'utilisation de particules magnétiques pour des applications dans les biotechnologies, l'industrie pharmaceutique ou la médecine est devenue de plus en plus fréquente. Les particules magnétiques ont vu le jour il y a déjà une vingtaine d'années et connaissent aujourd'hui un essor particulier, intimement lié aux objectifs de miniaturisation des analyses.

Leur taille, allant du nanométrique au micrométrique, et la possibilité de les manier à l'aide d'un simple champ magnétique, en font un outil bien adapté à la manipulation et à l'identification de molécules biologiques. De nombreuses sociétés fournissent une large variété de microbilles à la surface desquelles il est possible de greffer spécifiquement toutes sortes de molécules d'intérêt.

Dans les applications actuelles, les particules magnétiques utilisées sont majoritairement superparamagnétiques, c'est-à-dire qu'elles n'ont pas de rémanence magnétique, que le champ interne est nul en l'absence de champ magnétique externe, ce qui limite en théorie les phénomènes d'agrégation.

Toutefois, la plupart des nanoparticules magnétiques commerciales n'ont pas une grande stabilité colloïdale, et forment des agrégats lors des analyses effectuées par leur intermédiaire.

Et comme indiqué plus haut, la présence d'agrégats de nanoparticules magnétiques est à l'origine de faux positifs qui limitent la sensibilité et la spécificité des tests de diagnostic. En particulier, le diagnostic précoce par capteur GMR est extrêmement sensible, les objets biologiques marqués magnétiquement pouvant être détectées un par un, mais cette sensibilité, ainsi que la spécificité des tests, sont dégradées par la formation d'agrégats non spécifiques, c'est-à-dire non spécifiquement liés à la cible biologique à l'étude mais uniquement agglutinés pour des raisons physico-chimiques.

La présente invention a pour but de fournir des nano- ou microparticules qui évitent les inconvénients précités.

Ainsi, un but de l'invention est de fournir des nano- ou microparticules magnétiques ayant une grande stabilité colloïdale, permettant de s'affranchir du problème d'agrégation, notamment lors des tests de diagnostic précoce les impliquant.

Un autre but de l'invention est de fournir nano- ou microparticules ayant un moment magnétique équivalent à celui des billes commerciales, tout en permettant de s'affranchir du problème d'agrégation rencontré avec ces billes commerciales.

Un autre but de l'invention est de fournir nano- ou microparticules monodisperses, par un procédé simple et peu coûteux, lesquelles peuvent être facilement fonctionnalisées, notamment pour la préparation et la mise en oeuvre de dispositifs susceptibles d'être détectés par des capteurs à magnétorésistance géante (capteurs GMR) en tant qu'outils de diagnostic biologique.

Ainsi, selon un premier aspect, l'invention concerne une nano- ou microparticule comprenant une matrice constituée de ou comprenant au moins un alcool polyvinylique (PVA), et, en dispersion dans cette dernière, de la ferrite.

Par « nanoparticule », on entend notamment, sauf mention contraire, une particule ayant une taille comprise de 1 à 1000 nm, en particulier de 50 à 500 nm, plus particulièrement de 100 à 350 nm.

Par « microparticule », on entend notamment, sauf mention contraire, une particule ayant une taille comprise de 1 à 100 µm, en particulier de 1 à 10 µm, plus particulièrement de 1 à 5 µm.

Par « ferrite », on entend en particulier un composé de formule Fe₃O₄.

Par « dispersion », on entend en particulier que la ferrite est répartie sous la forme d'une pluralité de particules dans la matrice de PVA.

Selon un mode de réalisation particulier, la masse moléculaire de l'alcool polyvinylique est comprise de 10000 à 150000, en particulier de 50000 à 90000.

Un PVA trop grand peut être susceptible d'augmenter la viscosité du milieu de formation de la particule, le rendant très hétérogène. Avec un PVA de masse moléculaire trop faible, il est éventuellement possible que les germes de Fe₃O₄ coalescent et l'on obtienne alors un mélange de particules de tailles variées qui sont susceptibles d'être au-dessus du micron.

Selon un mode de réalisation particulier, la matrice est dénuée de polyvinylpyrrolidone (PVP).

Par « dénué de PVP », on entend que la matrice ne comprend pas de PVP.

Selon un mode de réalisation particulier, l'alcool polyvinylique (PVA) est partiellement acétylé. Dans ce cas, il correspond à un acétate de polyvinyle (ou un poly(acétate de vinyle), ou PVAC) partiellement hydrolysé. Plus particulièrement, l'alcool polyvinylique (PVA) est acétylé à moins de 20% (c'est-à-dire que moins de 20% des groupes -OH du PVA sont acétylés), par exemple à moins de 10% ou à 10% environ, ce qui correspond à un poly(acétate de vinyle) hydrolysé à plus de 80% (c'est-à-dire que plus de 80% des groupes -OAc du PVAC sont hydrolysés), par exemple à plus de 90% ou à 90% environ.

Selon un autre mode de réalisation particulier, l'alcool polyvinylique (PVA) n'est acétylé.

Dans ce cas, il correspond à un acétate de polyvinyle (ou un poly(acétate de vinyle), ou PVAC) totalement hydrolysé.

Selon un mode de réalisation particulier, l'alcool polyvinylique est réticulé, par exemple notamment à la surface de la nano- ou microparticule, en particulier à l'aide d'un agent réticulant choisi parmi les composés suivants : acide borique, acides boroniques, esters d'acide borique, esters d'acide boronique, borax (Na₂B₄O₇.10H₂O), et aldéhydes, notamment parmi les composés de formules suivantes : B(OR)₃, RB(OH)₂, B(OH)₃, R-CHO, dans lesquelles R est choisi parmi les aryles et les hétéroaryles, et les composés comprenant au moins deux fonctions aldéhyde, en particulier les composés comprenant deux fonctions aldéhyde, plus particulièrement les composés de formule H-C(=O)-(CH₂)ₙ-C(=O)-H, avec n allant de 0 à 6, par exemple n = 0, 1, 2 ou 3, l'agent réticulant étant par exemple l'acide 3-quinoline boronique. Lorsque le PVA est réticulé, il est susceptible d'être insensible à la présence de mauvais solvant, tel que l'éthanol, notamment quant à son volume.

Selon un mode de réalisation particulier, la nano- ou microparticule selon la présente invention a une forme de sphère ou de sphéroïde.

Par « sphéroïde », on entend en particulier une particule dont la forme est proche de la sphère, mais non parfaitement sphérique, et notamment que toutes les dimensions de la particule sont comprises entre 0,9 et 1 fois la plus grande dimension de ladite particule.

Selon un mode de réalisation particulier, la nano- ou microparticule selon la présente invention a une taille comprise de 50 à 1000 nm, en particulier de 150 à 450 ou 500 nm.

Cette taille correspond notamment à une nano- ou microparticule dont le PVA est exempt de solvant, en particulier d'eau, c'est-à-dire par exemple séchée une nuit à 100°C.

Si nécessaire, la taille de la nano- ou microparticule peut être modifiée : elle peut être diminuée par ajout d'un mauvais solvant tel que l'éthanol, ou augmentée (par hydratation) à l'aide d'un bon solvant, tel que l'eau.

Par « taille », on entend notamment la plus grande dimension de la particule.

Selon un mode de réalisation particulier, le au moins un alcool polyvinylique est présent dans la nano- ou microparticule selon la présente invention à hauteur de 30 à 50 % en masse par rapport à la masse totale de ladite nano- ou microparticule.

Selon un mode de réalisation particulier, la nano- ou microparticule selon la présente invention a une densité comprise de 1,7 à 2,5 g.cm⁻³, la densité étant par exemple d'environ 1,87 g.cm⁻³.

Selon un mode de réalisation particulier, la ferrite est sous la forme de cristallites.

Par « cristallites », on entend notamment des domaines de matière ayant la même structure qu'un monocristal.

Selon un mode de réalisation particulier, la ferrite est sous la forme de particules ayant une taille moyenne t_{A} comprise de 15 à 80 nm, notamment de 15 à 60 nm, plus particulièrement de 25 à 30 nm.

Par « taille », on entend notamment le diamètre de la particule lorsqu'elle sphérique, ou la plus grande dimension, lorsqu'elle ne l'est pas.

La taille moyenne peut par exemple être mesurée par analyse d'image réalisée par microscopie électronique à balayage (MEB).

Selon un mode de réalisation particulier, la ferrite est sous la forme de particules dont la distribution de taille a un coefficient d'uniformité compris de 0,95 à 1.

Selon un mode de réalisation particulier, la ferrite est sous la forme de particules dont plus de 95 % ont une taille 28 nm +/- 10%.

Selon un mode de réalisation particulier, la nano- ou microparticule selon la présente invention a un moment magnétique compris entre 3.10⁻¹⁵ et 5.10⁻¹¹Am².

Selon un autre aspect, la présente invention concerne également un ensemble de nano- ou microparticules telles que définie précédemment.

Tous les modes de réalisation décrits précédemment relativement à la nano- ou microparticule peuvent également s'appliquer ici, seuls ou en combinaison.

Selon un mode de réalisation particulier, la distribution de taille de l'ensemble de nano- ou microparticules de la présente invention a un coefficient d'uniformité compris de 0,95 à 1.

Selon un mode de réalisation particulier, la distribution de taille de l'ensemble de nano- ou microparticules de la présente invention est telle que plus de 95 % de ces nano- ou microparticules ont une taille 180 nm +/- 10%.

Selon un autre aspect, la présente invention concerne également un procédé de préparation d'une nano- ou microparticule telle que définie précédemment, ou d'un ensemble de nano- ou microparticule tel que défini précédemment, comprenant :
(i) une étape de mise en contact d'au moins un alcool polyvinylique (PVA) avec une composition A précurseur de ferrite pour obtenir une composition B;
(ii) une étape de chauffage de la composition B pour obtenir lesdites nano- ou microparticules.

Selon un mode de réalisation particulier, la composition A comprend un sel de fer (III), optionnellement hydraté.

Selon un mode de réalisation plus particulier, la composition A comprend un composé choisi parmi FeCl₃, Fe(NO₃)₃, Fe(ClO₄)₃, lesquels sont optionnellement hydratés, le composé étant de préférence choisi parmi FeCl₃, les sels Fe(NO₃)₃ hydratés, et les sels Fe(ClO₄)₃ hydratés.

En particulier, la composition A comprend ledit sel de fer (III), optionnellement hydraté, et est dénué de sel de fer (II), hydraté ou non.

Selon un mode de réalisation particulier, la composition A comprend de l'urée ou de l'hexaméthylènetétramine, en particulier de l'urée.

Selon un mode de réalisation particulier, la composition A comprend un chélatant hydrosoluble du fer.

Ledit chélatant hydrosoluble du fer est notamment choisi parmi l'acide citrique, les citrates, l'acide lactique, les lactates, l'acide tartrique, et les tartrates.

Selon un mode de réalisation particulier, la composition A comprend de l'urée ou de l'hexaméthylènetétramine, en particulier de l'urée, et un chélatant hydrosoluble du fer, en particulier choisi parmi l'acide citrique, les citrates, l'acide lactique, les lactates, l'acide tartrique, et les tartrates.

Selon un mode de réalisation particulier, l'étape (i) de mise en contact se fait en présence d'eau, la composition B obtenue à l'issue de ladite étape (i) étant en particulier une solution aqueuse.

Selon un mode de réalisation particulier, l'étape (ii) de chauffage se fait en autoclave.

Selon un mode de réalisation particulier, l'étape (ii) de chauffage se fait à une température comprise de 180 à 230 °C, et/ou pendant une durée comprise de 6 à 24 heures.

Selon un mode de réalisation particulier, postérieurement à l'étape (ii), et le cas échéant préalablement à l'étape (iii) telle que décrite plus bas, le au moins un alcool polyvinylique (PVA) est réticulé, en particulier à l'aide d'un agent réticulant choisi parmi les composés suivants : acide borique, acides boroniques, esters d'acide borique, esters d'acide boronique, borax (Na₂B₄O₇.10H₂O), et aldéhydes, notamment parmi les composés de formules suivantes : B(OR)₃, RB(OH)₂, B(OH)₃, R-CHO, dans lesquelles R est choisie parmi les aryles et les hétéroaryles, et les composés comprenant au moins deux fonctions aldéhyde, en particulier les composés comprenant deux fonctions aldéhyde, plus particulièrement les composés de formule H-C(=O)-(CH₂)ₙ-C(=O)-H, avec n allant de 0 à 6, par exemple n = 0, 1, 2 ou 3, l'agent réticulant étant par exemple l'acide 3-quinoline boronique.

Selon un autre aspect, la présente invention concerne également l'utilisation d'une nano- ou microparticule telle que définie précédemment, ou d'un ensemble de nano- ou microparticule tel que défini précédemment, pour la préparation de dispositifs aptes à être détectés par un capteur à magnétorésistance géante.

Dans tout ce qui suit, les modes de réalisation concernant une particule s'appliquent également à un ensemble de ces particules.

Selon un autre aspect, la présente invention concerne également une nano- ou microparticule comprenant une matrice constituée de ou comprenant au moins un alcool polyvinylique (PVA), et, en dispersion dans cette dernière, de la ferrite, et comprenant en outre une couche extérieure de silice.

Selon un mode de réalisation particulier, ladite couche extérieure de silice a une épaisseur comprise de 10 à 150 nm, notamment de 15 à 30 nm, ou de 30 à 150 nm.

La présence de cette couche extérieure de silice peut permettre d'éloigner les coeurs comprenant PVA et ferrite, ce qui est susceptible de conférer aux particules, si nécessaire, une stabilité avantageuse.

Selon un autre aspect, la présente invention concerne également un procédé de préparation d'une nano- ou microparticule telle que définie ci-dessus, comprenant :
(i) une étape de mise en contact d'au moins un alcool polyvinylique (PVA) avec une composition A précurseur de ferrite pour obtenir une composition B;
(ii) une étape de chauffage de la composition B pour obtenir lesdites nano- ou microparticules ;
(iii) une étape de dépôt d'une couche de silice sur la nano- ou microparticule telle qu'obtenue à l'issue de l'étape précédente par synthèse par voie sol gel, en particulier à l'aide d'un précurseur de silice choisi parmi le tetraéthylorthosilicate et le tetraméthylorthosilicate, optionnellement en présence d'aminopropyltriéthoxysilane.

Selon un autre aspect, la présente invention concerne également une nano- ou microparticule comprenant une matrice constituée de ou comprenant au moins un alcool polyvinylique (PVA), et, en dispersion dans cette dernière, de la ferrite, et comprenant en outre une couche extérieure de silice, laquelle porte à sa surface une couche d'Al(OH)₃.

Selon un mode de réalisation particulier, ladite couche d'Al(OH)₃ a une épaisseur comprise de 1 à 10 nm, notamment de 3 à 5 nm.

Selon un autre aspect, la présente invention concerne également une nano- ou microparticule comprenant une matrice constituée de ou comprenant au moins un alcool polyvinylique (PVA), et, en dispersion dans cette dernière, de la ferrite, et comprenant en outre une couche extérieure de silice, laquelle est fonctionnalisée par des composés portant des groupes acide carboxylique (-COOH) ou anhydride d'acide carboxylique (-C(=O)-O-C(=O)-), notamment des composés :
- constitués de ou comprenant un acide phosphonique dont le phosphore porte un alkyle en C₁-C₆ linéaire ou branché, substitué par un groupe acide carboxylique (-COOH) ;
- constitués de ou comprenant un arène portant deux anhydrides d'acide carboxylique (-C(=O)-O-C(=O)-), éventuellement hydrolysés.

Les composés comprenant un arène sont susceptibles d'être particulièrement rigides et donc d'éloigner le groupe acide carboxylique de la surface de la particule, ce qui peut permettre si nécessaire un couplage particulièrement efficient.

Selon un mode de réalisation particulier, la présente invention concerne une nano- ou microparticule comprenant une matrice constituée de ou comprenant au moins un alcool polyvinylique (PVA), et, en dispersion dans cette dernière, de la ferrite, et comprenant en outre une couche extérieure de silice, laquelle porte à sa surface une couche d'Al(OH)₃ fonctionnalisée en partie par des composés portant des groupes acide carboxylique (-COOH) ou anhydride d'acide carboxylique (-C(=O)-O-C(=O)-), notamment des composés :
- constitués de ou comprenant un acide phosphonique dont le phosphore porte un alkyle en C₁-C₆ linéaire ou branché, substitué par un groupe acide carboxylique (-COOH) ;
- constitués de ou comprenant un arène portant deux anhydrides d'acide carboxylique (-C(=O)-O-C(=O)-), éventuellement hydrolysés.

Selon un mode de réalisation particulier, la présente invention concerne une nano- ou microparticule comprenant une couche extérieure de silice, laquelle porte à sa surface une couche comprenant de l'aluminium, sous forme d'Al(OH)₃ ou formant un complexe de l'une des formules suivantes : 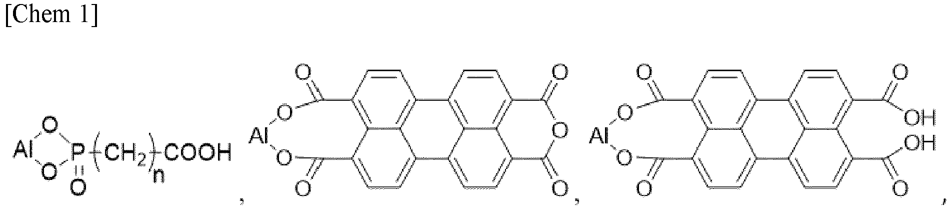 avec n allant de 1 à 6, en particulier 1 ou 2,
et leurs mélanges.

Selon un autre aspect, la présente invention concerne également un procédé de préparation d'une nano- ou microparticule telle que définie ci-dessus, comprenant :
(i) une étape de mise en contact d'au moins un alcool polyvinylique (PVA) avec une composition A précurseur de ferrite pour obtenir une composition B;
(ii) une étape de chauffage de la composition B pour obtenir lesdites nano- ou microparticules ;
(iii) une étape de dépôt d'une couche de silice sur la nano- ou microparticule telle qu'obtenue à l'issue de l'étape précédente par synthèse par voie sol gel, en particulier à l'aide d'un précurseur de silice choisi parmi le tetraéthylorthosilicate et le tetraméthylorthosilicate, optionnellement en présence d'aminopropyltriéthoxysilane ;
(iv) une étape de mise en contact d'une nano- ou microparticule telle qu'obtenue à l'issue de l'étape précédente avec un sel d'aluminium, notamment choisi parmi AlCl₃, Al(NO₃)₃, Al(ClO₄)₃, lesquels sont optionnellement hydratés, le sel d'aluminium étant de préférence choisi parmi AlCl₃, les sels Al(NO₃)₃ hydratés, et les sels Al(ClO₄)₃ hydratés, pour obtenir une nano- ou microparticule comprenant une couche extérieure de silice, laquelle porte à sa surface une couche d'Al(OH)₃ ;
(v) une étape de mise en contact de la nano- ou microparticule obtenue à l'issue de l'étape précédente avec un composé :
   ∘ constitué de ou comprenant un acide phosphonique dont le phosphore porte un alkyle en C₁-C₆ linéaire ou branché, substitué par un groupe acide carboxylique (-COOH) ;
   ∘ constitué de ou comprenant un arène portant deux anhydrides d'acide carboxylique (-C(=O)-O-C(=O)-) ;
   ∘ par exemple de formule suivante :

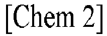

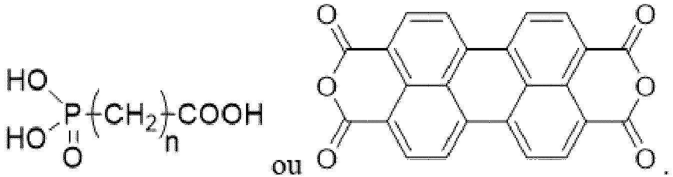

Selon un autre aspect, la présente invention concerne également une nano- ou microparticule comprenant une matrice constituée de ou comprenant au moins un alcool polyvinylique (PVA), et, en dispersion dans cette dernière, de la ferrite, et comprenant en outre une couche extérieure de silice, laquelle est fonctionnalisée par des composés portant une macromolécule, notamment un anticorps monoclonal,
ladite nano- ou microparticule comprenant notamment une couche extérieure de silice, laquelle est fonctionnalisée par des composés portant une macromolécule, notamment un anticorps monoclonal, par l'intermédiaire d'une fonction amide formée entre un acide carboxylique porté par lesdits composés et une amine portée par ladite macromolécule.

Selon un autre aspect, la présente invention concerne également un procédé de préparation d'une nano- ou microparticule telle que définie ci-dessus, comprenant :
(i) une étape de mise en contact d'au moins un alcool polyvinylique (PVA) avec une composition A précurseur de ferrite pour obtenir une composition B;
(ii) une étape de chauffage de la composition B pour obtenir lesdites nano- ou microparticules ;
(iii) une étape de dépôt d'une couche de silice sur la nano- ou microparticule telle qu'obtenue à l'issue de l'étape précédente par synthèse par voie sol gel, en particulier à l'aide d'un précurseur de silice choisi parmi le tetraéthylorthosilicate et le tetraméthylorthosilicate, optionnellement en présence d'aminopropyltriéthoxysilane ;
(iv) une étape de mise en contact d'une nano- ou microparticule telle qu'obtenue à l'issue de l'étape précédente avec un sel d'aluminium, notamment choisi parmi AlCl₃, Al(NO₃)₃, Al(ClO₄)₃, lesquels sont optionnellement hydratés, le sel d'aluminium étant de préférence choisi parmi AlCl₃, les sels Al(NO₃)₃ hydratés, et les sels Al(ClO₄)₃ hydratés, pour obtenir une nano- ou microparticule comprenant une couche extérieure de silice, laquelle porte à sa surface une couche d'Al(OH)₃ ;
(v) une étape de mise en contact de la nano- ou microparticule obtenue à l'issue de l'étape précédente avec un composé :
   ∘ constitué de ou comprenant un acide phosphonique dont le phosphore porte un alkyle en C₁-C₆ linéaire ou branché, substitué par un groupe acide carboxylique (-COOH) ;
   ∘ constitué de ou comprenant un arène portant deux anhydrides d'acide carboxylique (-C(=O)-O-C(=O)-) ;
   ∘ par exemple de formule suivante : 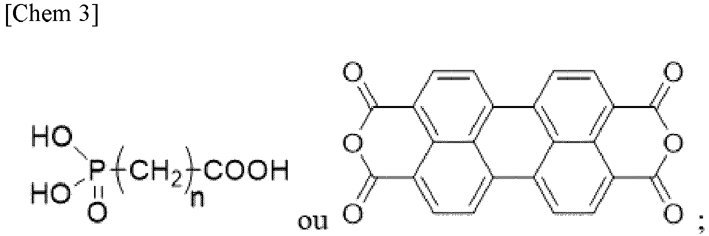
(vi) une étape de formation d'une fonction amide entre les groupes acide carboxylique portés par la nano- ou microparticule telle qu'obtenue à l'issue de l'étape précédente et une amine portée par ladite macromolécule, notamment par la formation d'ester activés intermédiaires à partir desdits acides carboxyliques, par exemple en utilisant du 1-éthyl-3-(3-diméthylaminopropyl)carbodiimide (EDC).

Selon un mode de réalisation particulier, l'étape (vi) est réalisée à un pH compris de 4 à 5, notamment de 4,5 environ.

Selon un mode de réalisation particulier, les procédés de préparation tels que décrits précédemment sont partiellement ou totalement réalisées en l'absence de champs magnétique.

Plus particulièrement, les étapes décrites ci-dessus, en particulier l'étape (ii) telle que décrite plus haut est (sont) réalisée(ées) en l'absence de champs magnétique.

Par « absence de champs magnétique », on entend notamment le fait de ne pas approcher d'aimant, notamment de ne pas utiliser d'agitation magnétique ni de dispositifs magnétisés comme un autoclave magnétisé.

Selon un autre aspect, la présente invention concerne également une nano- ou microparticule telle que définie ci-dessus, ou un ensemble de nano- ou microparticule telles que définies ci-dessus, pour son utilisation dans le diagnostic, notamment pour la détection de bactéries, en particulier dans des échantillons cliniques sans préculture, par exemple du sang total ou d'autres fluides biologiques ; ou de cellules cancéreuses.

Les bactéries sont notamment des bactéries pathogènes bien connues de l'homme du métier, en particulier d'intérêt environnemental et/ou de santé publique, par exemple *Yersinia enterocolitica* (susceptible d'être notamment responsable de gastroentérites, d'intoxications alimentaires) et *Legionella pneumophila* (susceptible d'être notamment responsable de pneumonies par inhalation de microgouttes).

### DEFINITIONS

Tel qu'on l'utilise dans la présente description, le terme « environ » se réfère à un intervalle de valeurs de ± 10 % d'une valeur spécifique. A titre d'exemple, l'expression « environ 20 » comprend les valeurs de 20 ± 10 %, soit les valeurs de 18 à 22.

Au sens de la présente description, les pourcentages se réfèrent à des pourcentages en masse par rapport à la masse totale de la formulation, sauf indication contraire.

Tel qu'on l'entend ici, les plages de valeur sous forme de « x-y » ou « de x à y » ou « entre x et y » incluent notamment les bornes x et y ainsi que les entiers compris entre ces bornes. A titre d'exemple, « 1-5 », ou « de 1 à 5 » ou « entre 1 et 5 » désignent notamment les entiers 1, 2, 3, 4 et 5. Les modes de réalisations préférés incluent chaque entier pris individuellement dans la plage de valeur, ainsi que toute sous-combinaison de ces entiers. A titre d'exemple, les valeurs préférées pour « 1-5 » peuvent comprendre les entiers 1, 2, 3, 4, 5, 1-2, 1-3, 1-4, 1-5, 2-3, 2-4, 2-5, etc.

Tel qu'il est utilisé ici, le terme "alkyle" désigne un groupe alkyle à chaîne linéaire ou ramifiée ayant le nombre d'atomes de carbone indiqué avant ledit terme, notamment 2 à 6 atomes de carbone, tel que éthyle, propyle, isopropyle, butyle, isobutyle, sec-butyle, tert-butyle, pentyle, isoamyle, néopentyle, 1-éthylpropyle, 3-méthylpentyle, 2,2-diméthylbutyle, 2,3-diméthylbutyle, hexyle, etc. Ainsi, une expression telle que "alkyle en C1-C4" désigne un radical alkyle contenant de 1 à 4 atomes de carbone. Il en est de même pour le terme « alcane ».

Les cycloalkyles sont en particulier des alkyles (tels que définis précédemment) comportant un cycle. Il s'agit par exemple du cyclohexyle.

Tel qu'utilisé ici, le terme "arène" désigne un système cyclique aromatique hydrocarboné mono- ou bicyclique, substitué ou non substitué, ayant 6 à 32 atomes de carbone dans le cycle. Les exemples incluent le benzène, naphtalène, anthracène, perylène, chryène, corannulène, phénanthrène, triphénylène, benzo[a]pyrène, coronène, tétracène, pentacène, pyrène, ovalène. Les arènes préférés comprennent le benzène, le naphtalène, le perylène, non substitués ou substitués. Sont inclus dans la définition d'"arène" les systèmes cycliques condensés, y compris, par exemple, les systèmes cycliques dans lesquels un cycle aromatique est condensé à un cycle cycloalkyle. Les exemples de tels systèmes cycliques condensés comprennent, par exemple, l'indane, l'indène et le tétrahydronaphtalène.

Tel qu'il est utilisé ici, le terme "hétéroarène" désigne notamment un groupe aromatique contenant 5 à 10 atomes de carbone dans le cycle, dans lequel un ou plusieurs atomes de carbone du cycle sont remplacés par au moins un hétéroatome tel que -O-, -N- ou -S-. Parmi les exemples de groupes hétéroarène, on peut citer les groupes pyrrole, furane, thiène, pyrazole, imidazole, thiazole, isothiazole, isoxazole, oxazole, oxathiole, oxadiazole, triazole, oxatriazole, furazane, tétrazole, pyridine, pyrazine, pyrimidine, pyridazine, triazine, indole, isoindole, indazole, benzofurane, isobenzofurane, purine, quinazoline, quinole, isoquinole, benzimidazole, benzothiazole, benzothiophène, thianaphtène, benzoxazole, benzisoxazole, cinnoline, phtalazine, naphtyridine et quinoxaline. La définition du terme "hétéroarène" inclut les systèmes cycliques fusionnés, y compris, par exemple, les systèmes cycliques dans lesquels un cycle aromatique est fusionné à un cycle hétérocycloalkyle. Des exemples de tels systèmes cycliques fusionnés comprennent, par exemple, le phtalamide, l'anhydride phtalique, l'indoline, l'isoindoline, la tétrahydroisoquinoline, le chromane, l'isochromane, le chromène et l'isochromène.

### FIGURES

[Fig 1] correspond à un cliché de microscopie électronique à balayage (MEB) des particules obtenues à l'exemple 1, sèches, telles qu'obtenues par dépôt d'une solution aqueuse desdites particules (solution mère) sur une plaque de silicium, puis analyse MEB.
[Fig 2] correspond à un cliché de microscopie électronique à balayage (MEB) des particules obtenues à l'exemple 1, à une échelle plus faible que celle de la figure 1.

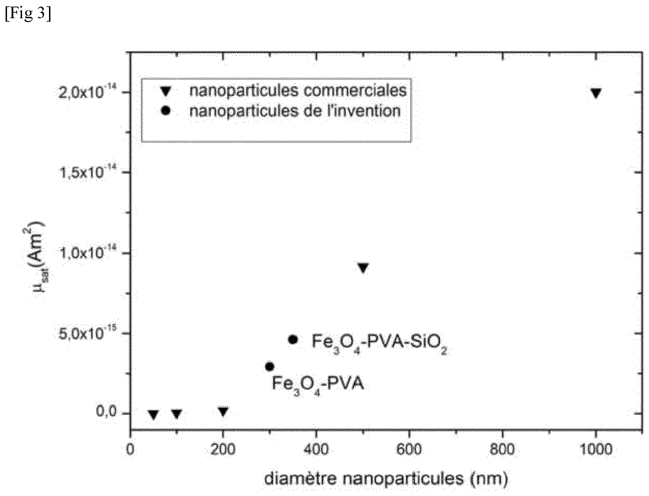

[Fig 3] est relative à l'extraction du moment magnétique de particules commerciales (billes micromod 50nm, billes ademtech 100nm, ademtech 200nm billes ademtech 200nm, billes micromod 500nm et dyna-Myone 1000nm) et de particules selon l'invention (exemple 1 et exemple 3).

### EXEMPLES

### Exemple 1 : Synthèse de billes de Fe₃O₄-PVA

1g de PVA est ajouté à 100 ml d'eau DI. Le milieu réactionnel est chauffé à 80°C pendant 1 heure environ, jusqu'à complète dissolution du PVA. La solution est limpide, incolore. On laisse la température redescendre à 50°C puis on ajoute 4,065g de FeCl₃, 6H₂O. La dissolution est totale. On ajoute alors 4,05g de FeCl₃, 6H₂O. La solution est alors rouge translucide.

Parallèlement, dans un autre récipient, on ajoute à 150ml d'eau DI, 8,82g de citrate de sodium 2H₂O et 2,7g d'urée.

Les deux solutions sont mélangées à température ambiante pendant 10 minutes, la couleur du mélange est orange et le milieu trouble. On verse alors la suspension obtenue dans un autoclave chemisé téflon. On ajoute 50ml d'eau DI. Puis l'autoclave est chauffé à 200°C pendant 24H. Après 24H on laisse l'autoclave refroidir. Le milieu réactionnel est une suspension noire. On récupère par centrifugation le précipité qui est lavé 3 fois à l'eau DI, chaque lavage étant suivi d'un cycle de centrifugation.

On récupère environ 2,2g de matière sèche (après une nuit à 100°C en étuve ventilée). On reprend alors la poudre dans 100ml d'EtOH anhydre puis on centrifuge à nouveau. Le précipité est à nouveau séché une nuit à 100°C. On récupère 1,9g de poudre. L'absorption atomique de la poudre donne un titre massique de 34% en Fe. Ce qui correspond à un titre massique en Fe3O4 de 47,36%.

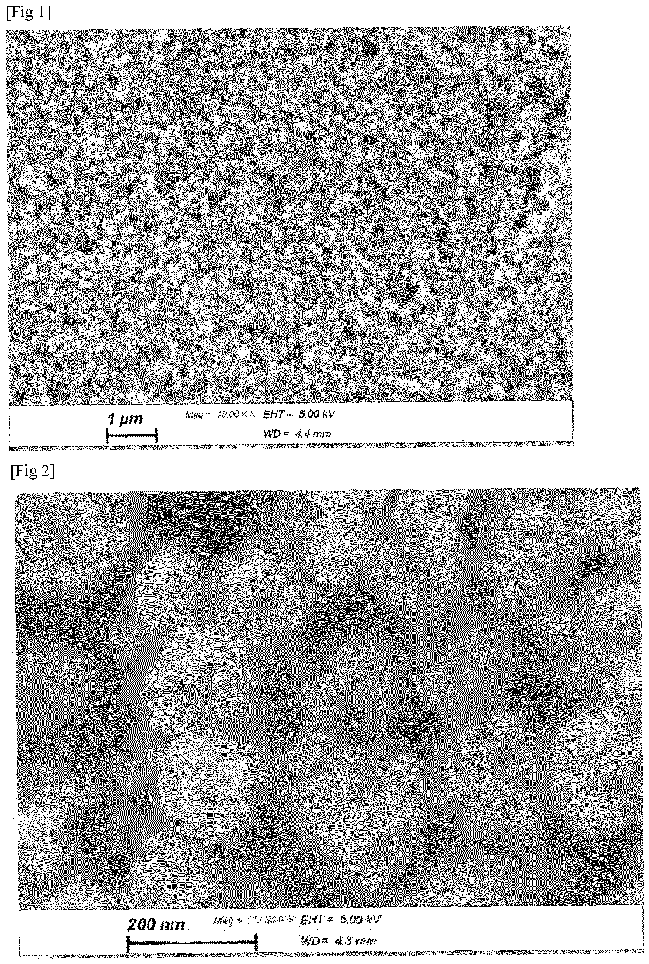

Les particules ainsi obtenues ont une densité de 1,87 kg.m⁻³ contre 5,17 kg.m⁻³ pour le Fe₃O₄. L'imagerie MEB (figure 1) permet de mettre en évidence la monodispersité en taille de particules composites de l'invention ainsi obtenues. Cette image montre également l'absence d'agrégation des particules de l'invention au sein de leur solution mère (un milieu d'usage biologique), et donc leur stabilité, particulièrement avantageuse, dans ledit milieu. En effet, s'il y avait eu agrégation en solution, celle-ci aurait également été observée après évaporation de la solution lors de la prise du cliché MEB.

L'imagerie MEB permet également de mettre en évidence la monodispersité en taille des particules de ferrite dans une particule composite de l'invention (figure 2).

### Exemple 2 : Propriétés magnétiques des particules de l'invention

Les particules magnétiques ont été caractérisées à l'aide de mesure d'aimantation en fonction du champ magnétique à température ambiante. Il a ainsi été possible de déterminer le moment magnétique d'une particule magnétique de l'invention et de le comparer à celui des particules magnétiques commerciales de taille équivalente (billes ademtech de 200 nm de diamètre et des billes micromod de 500 nm diamètre ). Le moment magnétique obtenu pour les particules magnétiques de l'invention (selon l'exemple 1 ou 3), ont un moment équivalent à celui des billes commerciales.

### Exemple 3 : Fonctionnalisation selon l'invention de particules de Fe₃O₄-PVA

On disperse sous agitation mécanique 1g de particules telles qu'obtenues dans l'exemple 1 dans 200ml d'EtOH abs. Dans ce milieu anhydre le PVA du composite est compacté. Puis on ajoute sous agitation 0,5g d'un mélange de tetraéthylorthosilicate et d'aminopropyltriéthoxysilane dans un rapport molaire TEOS/APTES =20/1 (l'APTES est susceptible de servir de catalyseur pour initier la polymérisation de la couche de silice). Après 10 minutes d'homogénéisation, on agite 1g d'H₂O. Le milieu est alors laissé sous agitation mécanique 12H à température ambiante. Le produit est récupéré par centrifugation, lavé trois fois à l'eau DI. Le potentiel zêta des particules passe à -20 -30 mV, illustrant que l'on a bien construit une couche de silice à la surface des particules.

Optionnellement, on peut réticuler le PVA du coeur Fe₃O₄-PVA en faisant réagir un réticulant du PVA : par exemple B(OR)₃, RB(OH)₂, B(OH)₃ ou Na₂B4O₇.10H₂O avant de faire croître la couche de silice. L'intérêt des composés tels que RB(OH)₂ est de pouvoir si désiré greffer une autre fonction organique sur le PVA de Fe₃O₄-PVA : notamment un colorant fluorescent.

L'acide 3-quinoline boronique a ainsi été utilisé avec succès : on disperse sous agitation mécanique 1g de particules Fe₃O₄-PVA dans 200ml d'H₂0 DI. Puis on ajoute sous agitation 0,2g d'acide 3-quinoline boronique dans 50ml de THF. Le milieu est alors laissé sous agitation mécanique 12H à température ambiante. Le produit est récupéré par centrifugation, lavé trois fois à l'eau DI, chaque lavage étant suivi d'une centrifugation. Le produit montre une fluorescence à 540nm lorsqu'il est excité à 400nm.

Les particules magnétiques peuvent porter des fonctions acides carboxyliques de surface, pouvant alors réagir, par exemple avec de l'EDC qui permettra le couplage, avec une molécule biologique.

Pour ce faire, on peut changer le potentiel zêta de la couche de silice en hydrolysant un sel d'aluminium (par exemple AlCl₃, Al(NO₃)₃). Sans vouloir se limiter à une quelconque théorie, l'aminopropyltriethoxysilane utilisé comme catalyseur est susceptible de participer au réseau de silice en laissant des fonctions amines primaires pendantes sur lesquelles les sels d'aluminium peuvent s'hydrolyser pour former une couche d'Al(OH)₃.

On disperse sous agitation mécanique 1g de particules PVA-Fe₃O₄ modifiées SiO₂ dans 200ml d'H₂0 DI. Puis on ajoute sous agitation 0,3g d'un sel d'aluminium hydraté. Le milieu est alors laissé sous agitation mécanique 12H à température ambiante. Le produit est récupéré par centrifugation, lavé trois fois à l'eau DI. Le potentiel zêta des particules passe à +15mV, prouvant que l'on a bien construit une couche d'Al(OH)₃ à la surface des particules. Sans vouloir se limiter à une quelconque théorie, la couche de silice amorphe de surface est susceptible de permet d'inhiber la formation de gibbsite cristallisée (Al(OH)₃. Sans cette couche, les cristaux de gibbsite pourraient commencer à croître à côté des particules de Fe₃O₄-PVA.

La couche de surface est ensuite fonctionnalisée par des greffons acides phosphoniques fonctionnels, afin que les particules magnétiques portent en surface des fonctions acides carboxyliques pendantes.

Pour ce faire, on disperse sous agitation mécanique 1g de particules Fe₃O₄-PVA modifiées SiO₂ à surface Al(OH)₃ dans 200ml d'H2O DI. Puis on ajoute sous agitation 0,2g de HOOCCH₂PO(OH)₂ et/ou HOOC(CH₂)₂PO(OH)₂. Le milieu est alors laissé sous agitation mécanique 12H à température ambiante. Le produit est récupéré par centrifugation, lavé trois fois à l'eau DI. Le potentiel zêta des particules passe à -10mV, prouvant que l'on a bien passivé une partie de la couche d'Al(OH)₃ à la surface des particules.

Alternativement, la couche de surface peut être fonctionnalisée par des polyanhydrides carboxyliques aromatiques. Par exemple, la réaction d'un dianhydride d'acides carboxyliques aromatiques comme le perylène-3,4,9,10-tetracarboxylic dianhydride sur les Al-OH de surface conduit à la formation du complexe suivant :

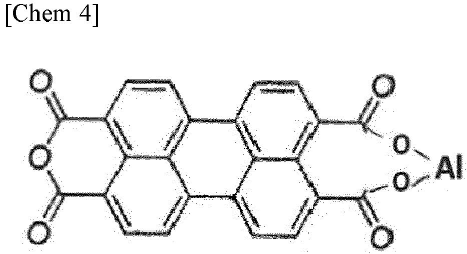

Il s'agit d'un bidente très solidement attaché à la surface Al(OH)₃.

Si le pH de la suspension colloïdale devient acide entre 4 et 5, la deuxième fonction anhydride s'ouvre formant le complexe suivant qui porte deux fonctions acides carboxyliques pendantes au bout d'un segment rigide sp².

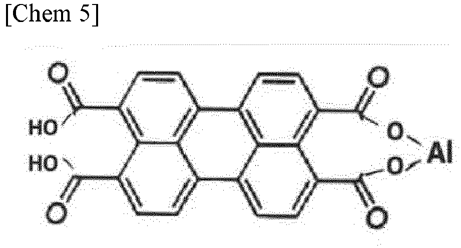

Ce greffon est fluorescent (exc 420nm donc dans les basses énergies, fluorescence dans le rouge). De plus, quand les groupements carboxyliques pendants réagissent, la fluorescence varie. C'est donc une sonde qui permet d'évaluer la qualité du couplage EDC tel que décrit ci-dessous.

Pour ce faire, on disperse sous agitation mécanique 1g de particules Fe₃O₄-PVA modifiées SiO₂ à surface Al(OH)₃ dans 200ml d'H₂O DI. Puis on ajoute sous agitation 0,1g de perylène-3,4,9,10-tetracarboxylique dianhydride dans 15ml de THF. Le milieu est alors laissé sous agitation mécanique 12H à température ambiante. Le produit est récupéré par centrifugation, lavé trois fois à l'eau DI. Le potentiel zêta des particules passe à -5 mV, prouvant que l'on a bien passivé une partie de la couche d'Al(OH)₃ à la surface des particules. Les particules sont rouges.

Pour fonctionnaliser les billes magnétiques, les fonctions carboxyliques de surface sont dans un premier temps modifiées en esters activés par réaction à l'EDC. Puis, dans un second temps, en présence des amines primaires des chaînes latérales des anticorps, une liaison amide est formée, assurant ainsi le greffage covalent des anticorps à la surface des billes magnétiques.

Pour cela, à chaque milligramme de billes magnétiques, on ajoute 80 µL d'une solution d'EDC (1-éthyl-3-(3-diméthylaminopropyl) carbodiimide) préparée extemporanément à 4 mg/mL en tampon MES 0.1 M pH4.7. Le mélange est incubé 10 mn à 37°C sous agitation à 150 rpm pour obtenir les esters activés intermédiaires à partir des acides carboxyliques.

Ensuite, 10 à 50 µg d'anticorps purifié sont ajoutés par mg de billes pour réaction de conjugaison pendant 2 h à 37°C sous agitation à 150 rpm. Enfin, 2 mL de BSA à 0,5 mg/mL en tampon MES 0.1 M pH4.75 sont ajoutés par mL de billes fonctionnalisées. Le mélange est incubé une dernière fois 30 mn à 37°C sous agitation à 150 rpm pour assurer la saturation des sites non spécifiques ou n'ayant pas réagi. Les billes magnétiques ainsi fonctionnalisées sont lavées deux fois par centrifugation en PBS pH7.4 + 0.1% BSA et reprises dans ce dernier tampon à une concentration finale de 5 mg/mL pour un stockage à 4°C jusqu'à utilisation.

## Revendications

1. Nano- ou microparticule comprenant une matrice constituée de ou comprenant au moins un alcool polyvinylique (PVA), et, en dispersion dans cette dernière, de la ferrite.

2. Nano- ou microparticule selon la revendication 1, dans laquelle l'alcool polyvinylique est réticulé, en particulier à l'aide d'un agent réticulant choisi parmi les composés suivants : acide borique, acides boroniques, esters d'acide borique, esters d'acide boronique, borax (Na₂B₄O₇.10H₂O), et aldéhydes, notamment parmi les composés de formules suivantes : B(OR)₃, RB(OH)₂, B(OH)₃, R-CHO, dans lesquelles R est choisie parmi les aryles et les hétéroaryles, et les composés comprenant au moins deux fonctions aldéhyde, en particulier les composés comprenant deux fonctions aldéhyde, plus particulièrement les composés de formule H-C(=O)-(CH₂)ₙ-C(=O)-H, avec n allant de 0 à 6, par exemple n = 0, 1, 2 ou 3, l'agent réticulant étant par exemple l'acide 3-quinoline boronique.

3. Nano- ou microparticule selon l'une quelconque des revendications précédentes, laquelle :
- a une forme de sphère ou de sphéroïde ; et/ou
- a une taille comprise de 50 à 1000 nm, en particulier de 150 à 450 ou 500 nm.

4. Nano- ou microparticule selon l'une quelconque des revendications précédentes, dans laquelle le au moins un alcool polyvinylique y est présent à hauteur de 30 à 50 % en masse par rapport à la masse totale de ladite nano- ou microparticule.

5. Ensemble de nano- ou microparticules selon l'une quelconque des revendications précédentes, dont la distribution de taille a un coefficient d'uniformité compris de de 0,95 à 1 et/ou dont plus de 95 % de ces nano- ou microparticules ont une taille 180 nm +/- 10%.

6. Procédé de préparation de nano- ou microparticule selon l'une quelconque des revendications 1 à 4, ou d'un ensemble de nano- ou microparticule selon la revendication 5, comprenant :
(i) une étape de mise en contact d'au moins un alcool polyvinylique (PVA) avec une composition A précurseur de ferrite pour obtenir une composition B;
(ii) une étape de chauffage de la composition B pour obtenir lesdites nano- ou microparticules.

7. Utilisation d'une nano- ou microparticule selon l'une quelconque des revendications 1 à 4, ou d'un ensemble de nano- ou microparticule selon la revendication 5, pour la préparation de dispositifs aptes à être détectés par des capteurs à magnétorésistance géante.

8. Nano- ou microparticule selon l'une quelconque des revendications 1 à 4, comprenant en outre une couche extérieure de silice, laquelle est fonctionnalisée par des composés portant une macromolécule, notamment un anticorps monoclonal,
ladite couche extérieure de silice étant notamment fonctionnalisée par l'intermédiaire d'une fonction amide formée entre un acide carboxylique porté par lesdits composés et une amine portée par ladite macromolécule.

9. Procédé de préparation d'une nano- ou microparticule selon la revendication 8 comprenant :
(iii) une étape de dépôt d'une couche de silice sur la nano- ou microparticule selon l'une quelconque des revendications 1 à 4 par synthèse par voie sol gel, en particulier à l'aide d'un précurseur de silice choisi parmi le tetraéthylorthosilicate et le tetraméthylorthosilicate, optionnellement en présence d'aminopropyltriéthoxysilane ;
(iv) une étape de mise en contact de la nano- ou microparticule obtenue à l'issue de l'étape précédente avec un sel d'aluminium, notamment choisi parmi AlCl₃, Al(NO₃)₃, Al(ClO₄)₃, lesquels sont optionnellement hydratés, le sel d'aluminium étant de préférence choisi parmi AlCl₃, les sels Al(NO₃)₃ hydratés, et les sels Al(ClO₄)₃ hydratés, pour obtenir une nano- ou microparticule comprenant une couche extérieure de silice, laquelle porte à sa surface une couche d'Al(OH)₃ ;
(v) une étape de mise en contact de la nano- ou microparticule obtenue à l'issue de l'étape précédente avec un composé :
∘ constitué de ou comprenant un acide phosphonique dont le phosphore porte un alkyle en C₁-C₆ linéaire ou branché, substitué par un groupe acide carboxylique (-COOH) ;
∘ constitué de ou comprenant un arène portant deux anhydrides d'acide carboxylique (-C(=O)-O-C(=O)-) ;
∘ par exemple de formule suivante : 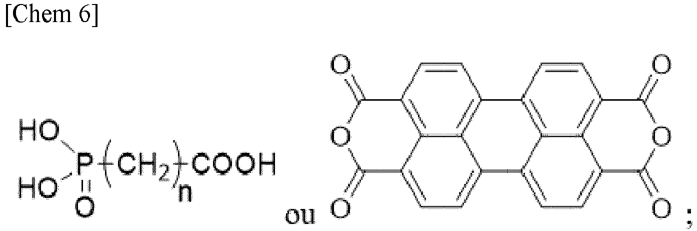
(vi) une étape de formation d'une fonction amide entre les groupes acide carboxylique portés par la nano- ou microparticule obtenue à l'issue de l'étape précédente et une amine portée par ladite macromolécule, notamment par la formation d'ester activés intermédiaires à partir desdits acides carboxyliques, par exemple en utilisant du 1-éthyl-3-(3-diméthylaminopropyl)carbodiimide (EDC).

10. Nano- ou microparticule selon la revendication 8 pour son utilisation dans le diagnostic, notamment pour la détection de bactéries, en particulier dans des échantillons cliniques sans préculture, par exemple du sang total ou d'autres fluides biologiques ; ou de cellules cancéreuses.
